## Europäisches Patentamt
## European Patent Office
## Office européen des brevets

(19)

(11) Numéro de publication: **0 140 790**
**B1**

(12)

# FASCICULE DE BREVET EUROPÉEN

(45) Date de publication du fascicule du brevet:
29.03.89

(51) Int. Cl.⁴: **A 61 F 5/02,** A 61 B 17/58

(21) Numéro de dépôt: **84402149.3**

(22) Date de dépôt: **24.10.84**

---

(54) **Appareil de correction dynamique des déformations rachidiennes.**

---

(30) Priorité: **28.10.83 FR 8317289**

(43) Date de publication de la demande:
**08.05.85 Bulletin 85/19**

(45) Mention de la délivrance du brevet:
**29.03.89 Bulletin 89/13**

(84) Etats contractants désignés:
**BE DE GB IT LU NL SE**

(56) Documents cité:
**DE-A-3 032 237**
**FR-A-2 275 679**
**FR-A-2 516 788**
**GB-A-780 652**
**US-A-4 289 123**

(73) Titulaire: **Peze, William, 16, rue de Jemmapes,
F-78800 Houilles (FR)**

(72) Inventeur: **Peze, William, 16, rue de Jemmapes,
F-78800 Houilles (FR)**

(74) Mandataire: **Mongrédien, André, c/o SOCIETE DE
PROTECTION DES INVENTIONS 25, rue de
Ponthieu, F-75008 Paris (FR)**

EP 0 140 790 B1

LIBER, STOCKHOLM 1989

## Description

La présente invention a pour objet un appareil de correction des déformations rachidiennes et essentiellement de la scoliose qui est la cause majeure de telles déformations.

Cet appareil est destiné à être implanté chirurgicalement le long de la partie déformée du rachis d'un enfant en cours de croissance et à y rester quelques mois ou années pendant lesquelles il exerce sur le profil de ce rachis une action mécanique correctrice destinée à faire regresser la déformation initiale. Lorsque la croissance est terminée ou lorsque la correction obtenue est satisfaisante, on intervient une seconde fois chirurgicalement pour retirer l'appareil.

La scoliose est une maladie déformante du rachis qui atteint un ou plus rarement deux segments de celui-ci en période de croissance. Cette déformation est particulièrement grave car, du fait qu'elle associe une torsion horizontale et une flexion dans un plan frontal, elle se développe dans les trois dimensions de l'espace. C'est par ailleurs une maladie évolutive qui prend naissance au cours d'une poussée de roissance, vraisemblablement à la suite de la rotation d'un ou de deux corps vertébraux. La scoliose est avant tout une affection dynamique évolutive et plusieurs tests connus (notamment le "Bending test") montrent son caractère corrigible, au moins en partie, au début de l'affection. Par la suite, la déformation réalisée a tendance à se fixer par enraidissement des pièces articulaires et déformations des pièces osseuses. Il est donc important de tenter une correction de cette déformation le plus tôt possible après la mise en évidence de son apparition.

Les traitements actuellement connus de la scoliose sont d'abord orthopédiques et dynamiques (port de corset rigide, traction exercée sur la colonne vertébrale notamment par suspension du malade par la tête, kinésithérapie). Si le traitement ainsi entrepris est insuffisant et que l'on assiste à une aggravation de la déformation, on recourt alors à des interventions chirurgicales. Ces derniers traitements ont pour but, après avoir réduit au maximum les déformations de fixer les lésions au moyen de matériels rigides et d'arthrodèses pour éviter l'aggravation de la déformation. Ces méthodes qui sont parfois nécessaires pour éviter le pire, ont néanmoins le grave inconvénient d'être définitives, et de pérenniser la déformation en bloquant la croissance.

Il existe également des méthodes de traitement des déformations de la colonne vertébrale par implantation chirurgicale, le long de la partie malade, d'une structure métallique flexible de redressement, que l'on place contre les apophyses épineuses. C'est le cas notamment du dispositif implantable décrit dans le brevet français FR-A-2 516 788 et qui se compose essentiellement d'une lame unique, ayant vraisemblablement une position au repos simplement rectiligne (n'ayant donc pas a priori la mémoire de forme d'un rachis normal), et qui est simplement fixée à ses deux extrémités et appuyée contre la colonne le long des apophyses épineuses. Il en résulte plusieurs constatations qui permettent de déceler certains défauts graves, inhérents à la structure de ce dispositif.

D'abord, et ceci est important, une telle lame ne peut fournir d'efforts de rappel que dans le sens de sa flexion propre, c'est-à-dire dans un plan unique et non pas dans toutes les directions de l'espace comme cela est souhaitable pour une correction efficace.

Ensuite, du fait qu'elle n'est pas fixée localement à chaque vertèbre, elle est incapable d'exercer une action correctrice segmentaire sud la partie du rachis malade, en particulier une action antirotation qui est un des effets capitaux du dispositif objet de l'invention.

De plus, cette absence de fixation sur chaque vertèbre est la cause du fait qu'il existe un risque non négligeable de voir la tige s'échapper et glisser le long de la colonne au bout d'un certain temps d'application sur le malade.

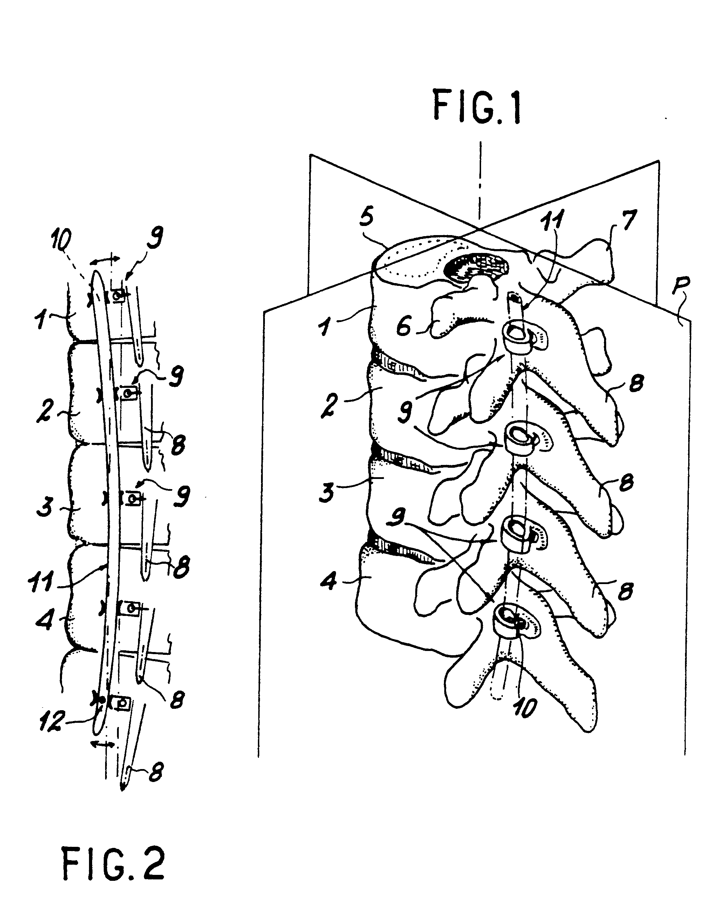

Par ailleurs, il est précisé dans le texte et sur la fig. 1, que la tige de rappel est fixée aux deux extrémités par un système à crémaillère assez compliqué, ce qui nécessite d'une part de réintervenir de temps en temps pour refaire le serrage et, d'autre part, interdit pratiquement un port prolongé par le malade sans intervention car la croissance du rachis ne peut pas se produire normalement pendant le temps d'implantation de la prothèse.

Enfin et surtout, la conception d'ensemble du dispositif implantable selon le brevet français FR-A-2 516 788 amène à exercer sur la colonne malade des efforts de redressement particulièrement intenses (400 à 600 N comme indiqué page 11, lignes 14 et 15) qui ne sont pas sans inconvénients sérieux pour le patient.

La présente invention a pour objet un appareil de correction dynamique des déformations rachidiennes, et notamment des scolioses qui permet une cure de celles-ci par l'action douce et permanente d'un appareil de correction implanté chirurgicalement.

Cet appareil de correction dynamique des déformations rachidiennes, comprenant une structure flexible en matériau biocompatible de forme générale allongée et destinée à être implantée le long de la partie du rachis que l'on cherche à corriger, se caractérisé en ce que cette structure flexible est une structure élastique de rappel ayant au repos la forme de la partie correspondante d'un rachis normal et apte à exercer, lorsqu'on l'implante sur la partie malade du rachis, une correction d'intensité légère et constante dans les trois directions de l'espace, à savoir une torsion dans un plan frontal, une torsion dans un plan saggital et un couple de torsion selon l'axe de la colonne, ladite structure étant composée d'au moins une tige élastique, fixable sur chaque vertèbre (1, 2, 3, 4) de la partie malade à l'aide de brides de retenue (9, 17) vissables dans ces mêmes vertèbres (1, 2, 3, 4) dans l'angle formé par l'apophyse épineuse (8) et la lame, chaque bride de retenue (9, 17) comportant au moins un orifice de guidage (10, 19) la traversant de part en part parallèlement à l'axe du rachis, ladite tige élastique étant enfilée dans les orifices de guidage (10, 19) et immobilisée en rotation dans chaque orifice de guidage et en translation sur une seule des brides de retenue.

La caractéristique essentielle de l'appareil de correction dynamique objet de l'invention est donc de posséder une structure élastique de rappel douée d'une mémoire de forme, correspondant à un rachis au profil normal et qui vient ainsi appliquer au segment rachidien malade une tension et un couple permanents et doux visant à corriger sa deformation. Il est certain, et ceci est capital, que le caractère permanent d'une action douce est infiniment plus efficace, pour obtenir au moins partiellement une correction du profil du rachis et éviter l'aggravation d'une scoliose, qu'une action violente et brutale appliquée pendant un temps limité. L'action douce résultant de l'effort permanent de la structure élastique de rappel gardant en mémoire la forme correcte dans les trois dimensions de la partie correspondante d'un rachis normal s'applique ainsi contre toutes les composantes qui entraînent la déformation scoliotique et avant tout la rotation ainsi que les inflexions dans le plan frontal.

L'ensemble de l'appareil implantable doit être évidemment réalisé en un matériau biocompatible. A titre d'exemple non limitatif, on peut choisir l'acier inoxydable NSM 21 S (norme Ugine). La longueur et la section de la structure élastique de rappel douée de mémoire sont étudiées en fonction de l'âge de l'individu soigné et des performances dynamiques que l'on souhaite obtenir. Elle est conçue pour être mise en place au fond de la fosse épineuse le long de la partie postérieure de chaque vertèbre malade et elle doit être bien entendu résistante à l'usure. Par ailleurs, le principe même de l'appareil objet de l'invention fait que la force correctrice et le couple de torsion appliqués par la structure élastique de rappel à la partie du rachis malade sont fonction directe du degré de déformation géométrique de ce rachis et augmentent avec lui, ce qui est un avantage très important.

L'appareil de correction objet de l'invention doit être utilisé plutôt à titre préventif que curatif et par conséquent mis en place le plus tôt possible dans le cours de la période évolutive de la maladie, lorsque les déformations du rachis sont encore souples et que les pièces osseuses n'ont pas encore subi de déformation. Il est donc nécessaire, pour choisir le bon moment d'application de l'appareil, objet de l'invention, de juger de façon très précoce de l'existence et du caractère évolutif d'une scoliose débutante.

On remarquera encore que les tiges de la structure élastique de rappel qui sont en quelque sorte l'élément actif de l'appareil de correction objet de l'invention, n'étant qu'enfilées de façon coulissante dans les orifices de guidage des brides de retenue, laissent entièrement libre le déplacement axial de chaque vertèbre et permettent par conséquent la croissance du rachis pendant tout le temps que dure l'implantation de l'appareil. Simplement, les orifices de guidage des brides de retenue ont une forme correspondant à la section droite des tiges élastiques qui exclut la rotation de celles-ci autour de leur axe pour permettre l'application d'efforts de torsion, qui ne pourraient avoir lieu si les tiges étaient capables de tourner sur elles-mêmes dans chacun des orifices de guidage. A cet effet et selon l'invention, les orifices de guidage ont une forme spéciale choisie notamment dans le groupe qui comprend les sections ovale, polyédrique, ou les rails et les croix. Lorsque l'orifice de guidage a une section droite ovale, le grand axe de cet ovale est contenu de préférence dans un plan sagittal. Enfin, les orifices de guidage des brides de retenue peuvent avoir à leur entrée comme a leur sortie une forme évasée qui permet une certaine flexion de la tige élastique entre les différents points de guidage.

Selon l'invention, chaque bride de retenue de la tige élastique est fixable à la vertèbre associée par tout moyen connu et notamment selon plusieurs modes de réalisation spécialement intéressants qui sont les suivants.

Selon un premier mode de réalisation, chaque bride de retenue est fixable à la vertèbre associée par vissage direct dans la lame contro-latérale.

Selon un deuxième mode de réalisation de l'invention, chaque bride de retenue est fixable à la vertèbre associée à l'aide d'une tige filetée traversant l'apophyse épineuse et serrée, sur l'autre face de celle-ci, par un écrou d'application d'un flasque d'appui qui vient se loger dans l'arc posterieur de la vertèbre.

Selon un troisième mode de réalisation de l'invention, chaque bride de retenue est fixable à la vertèbre associée par vissage direct dans le pédicule vertebral.

D'une façon générale, les trois modes de réalisation précédénts sont donnés à titre indicatif et non limitatif et il est bien entendu que le chirurgien en tant qu'homme de métier peut utiliser tout autre mode de fixation qui lui semblera approprié à chaque cas particulier, et notamment toute combinaison des trois cas précédénts, sans sortir du cadre de l'invention.

Les dispositions précédentes de l'appareil de correction, objet de l'invention, permettent d'appliquer la tension correctrice de la ou des tiges élastiques sur l'arc postérieur de chaque vertèbre qui est d'un accès aisé tout au long du rachis. D'une façon générale en effet, le point d'application de la force correctrice doit être situé le plus près possible du centre de rotation vertébral afin d'avoir le maximum d'efficacité en ce qui concerne les couples appliqués comme ce centre est situé dans le canal rachidien, il est impossible pour des raisons évidentes d'y appliquer un couple. La zone la plus proche et la plus facilement accessible par la voie dorsale est l'angle épineuse-lame et c'est donc sur cette zone précise que l'appareil de correction, objet de l'invention, vient appliquer son couple correcteur c'est également sur cette même zone que le même appareil exerce la force de rappel qui vise à corriger les courbures frontales pathologiques acquises par un rachis déformé.

De toute façon, l'invention sera mieux comprise à la lecture qui suit de la description de plusieurs exemples de mise en oeuvre de l'appareil de correction objet de l'invention, description qui sera faite à titre surtout illustratif et non limitatif et sera faite en se référant aux figures 1 à 9 ci-jointes, sur lesquelles:

- la figure 1 représente une vue générale en perspective de l'appareil de correction, objet de l'invention, dans le cas ou la structure élastique de rappel est constituée d'une seule tige élastique douée de mémoire de forme qui constitue la pièce active de l'appareil,
- la figure 2 représente, vue de côté, dans l'exemple de la figure 1, la tige élastique en place sur une portion déformée de rachis,
- la figure 3 montre un exemple de mise en oeuvre de l'invention dans le cas où la structure élastique de rappel est constituée de plusieurs tiges élastiques en parallèle,
- la figure 4 montre le détail de réalisation d'une des brides de retenue de l'exemple de la figure 3,
- la figure 5 montre la forme évasée des trous de section carrée de la bride de retenue de l'exemple de la figure 4,
- les figures 6, 7 et 8 illustrent différents modes possibles de fixation des brides de retenue sur les vertèbres correspondantes,
- la figure 9 illustre un mode particulier de réalisation de la structure de rappel élastique de l'appareil de correction, objet de l'invention.

Sur la figure 1, on a représenté quatre vertèbres 1, 2, 3 et 4 d'une portion de rachis atteint d'une déformation du type scoliose par rotation vers la gauche du dessin par rapport au plan sagittal P passant par l'axe de la vertèbre 1. On reconnaît sur chaque vertèbre le corps vertébral 5 et les apophyses transverses 6 et 7 et épineuses 8. Selon l'invention, des brides de retenue 9 dont la forme et la fixation sont expliquées plus en détail en se référant aux figures qui suivent, sont logées dans l'arc postérieur de la vertèbre entre l'apophyse épineuse 8 et l'apophyse transverse 6. Dans l'exemple particulier de la figure 1, la structure élastique de rappel est composée d'une tige unique 11 en un matériau élastique et biocompatible. Chaque bride de retenue 9 est munie d'un orifice 10 dans lequel passe la tige élastique 11 dotée d'une mémoire de forme. Sur la figure 1, la tige 11, pour plus de clarté, n'a été représentée que partiellement et en pointillés.

La tige élastique 11 a été conçue, préalablement à son implantation dans l'appareil de la figure 1, de façon à posséder la mémoire de forme d'une partie correspondant aux vertèbres 1, 2, 3 et 4 de la figure 1, d'un rachis normal et exerce de ce fait exactement la tension permanente et douce nécessaire pour ramener l'ensemble des vertèbres 1, 2, 3 et 4 par flexion et torsion dans leur position initiale correcte, c'est-à-dire pour corriger la position de l'ensemble rachidien représenté. Les orifices de guidage 10 ont une section droite identique à celle de la tige élastique 11 et notamment de forme ovale, de façon à empêcher toute rotation de la tige 11 autour de son axe dans les orifices 10, permettant ainsi l'action de torsion de cette tige 11 sur la partie de rachis équipée de l'appareil. Par ailleurs, chaque orifice de guidage 10 a éventuellement des extrémités supérieure et inférieure de forme évasée, de façon à permettre la flexion harmonieuse de la tige 11 entre ses différents points de liaison.

La tige 11 munie de ses différentes brides de retenue 9 est fixée par une vis pointeau 12 à l'une d'entre elles de façon à être bloquée en translation longitudinale. Tous les autres mouvements de la tige de torsion 11 sont ainsi permis et son passage a frottement doux dans les brides de retenue 9 autorise la croissance du rachis du sujet malade durant tout le temps d'implantation de l'appareil.

Sur la figure 1, la section droite des orifices de guidage 10 a été prévue de forme ovale ayant un grand axe situé dans un plan sagittal. Ceci n'est pas limitatif et cette section peut être de forme très variable telle que polyédrique ou en forme de rail ou de croix, seule la section circulaire étant interdite puisqu'elle permettrait la rotation de la tige 11 autour de son axe et annulerait toute possibilité d'effort en torsion de celle-ci sur le rachis.

Il faut noter enfin que la tige 11 ne nécessite une fixation en translation qu'au niveau d'une seule de ses brides de retenue afin d'éviter son glissement selon l'axe. Cette bride de retenue peut être par exemple l'une des brides d'extrémité supérieure ou inférieure de l'appareil et il suffit d'une petite vis la traversant pour serrer la tige une fois l'appareil mis en place.

Sur la figure 2, on a représenté une vue de profil de l'appareil de correction de la figure 1, sur laquelle on retrouve les quatre vertèbres 1, 2, 3, 4, la tige élastique 11 enfilée dans les orifices 10 de forme ovale évasée des différentes brides de retenue 9. On voit également en 12 la trace de la vis de fixation en translation de la tige 11 sur la dernière bride de retenue 9. Cette figure 2 permet de voir, entre autres choses, la courbure de la tige élastique 11 dans un plan sagittal.

Sur les figures 1 à 2, les orifices de guidage 10 ne sont pas rigoureusement cylindriques mais, conformément à l'invention, de forme évasée permettant une certaine flexion de la tige 11 entre deux brides de retenue 9 consécutives cette précaution est indispensable pour que la tige puisse jouer son rôle complet en fournissant sur le rachis l'appui nécessaire à la correction que l'on attend d'elle. De même, les orifices de guidage 10 ont une section ovale, dont le grand axe est situé dans un plan sagittal pour empêcher la rotation de la tige 11 sur elle-même et lui permettre d'exercer son couple de torsion sur chaque vertèbre.

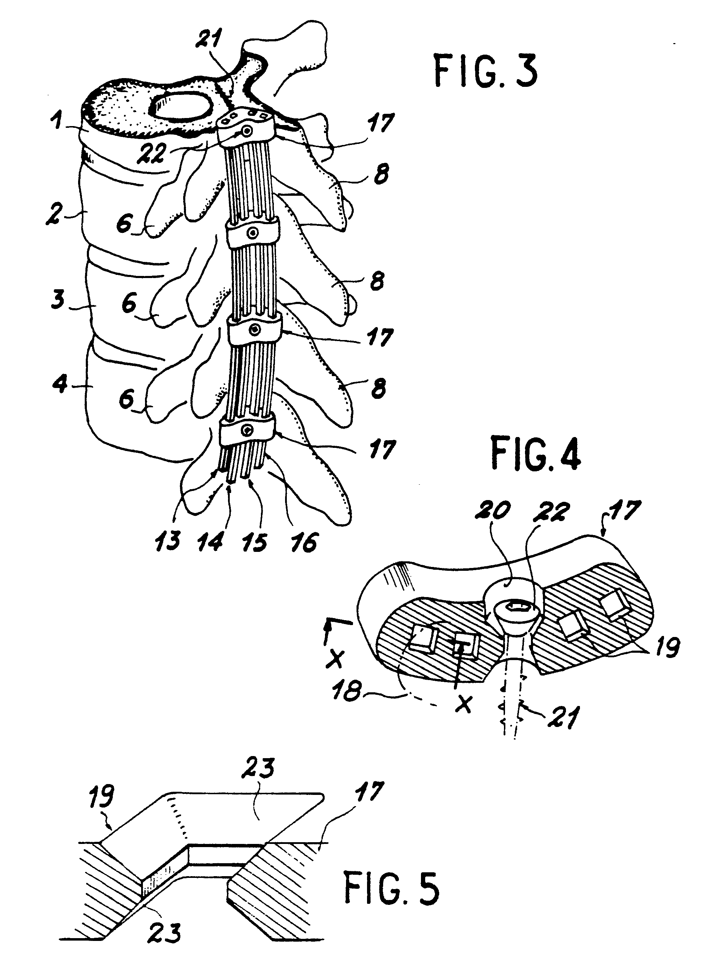

La figure 3 représente une variante très intéressante de l'appareil de la figure 1 dans laquelle la structure élastique de rappel est constituée d'un faisceau de quatre tiges élastiques en parallèle. On retrouve sur cette figure 3 les éléments de la figure 1 qui portent les mêmes nombres de référence. Simplement, la tige élastique unique de la figure 1 est remplacée par le faisceau des quatre tiges élémentaires 13, 14, 15, 16 de section carrée, maintenues en place dans l'angle épineuse-lame des vertèbres par des brides de retenue 17 comportant quatre orifices de section carrée dont chacun correspond à l'une des tiges élémentaires 13, 14, 15 et 16 et qui seront décrits plus en détail sur la figure 4.

Ce mode de réalisation est spécialement intéressant, car il permet d'utiliser des tiges élémentaires élastiques 13, 14, 15, 16 plus fines et dont l'assemblage ainsi constitué possède néanmoins toute l'élasticité et la souplesse nécessaires. Selon les cas, on peut utiliser soit des tiges élémentaires 13, 14, 15, 16 purement et simplement rectilignes, soit des tiges plus ou moins préformées au profil d'une portion de rachis correspondante et normale. Comme dans l'exemple de la figure 1, la structure de rappel ainsi constituée exerce sur chaque vertèbre de façon permanente, une force et un couple de faible intensité, ce qui est le résultat fondamental recherché pour obtenir une correction progressive dans de bonnes conditions.

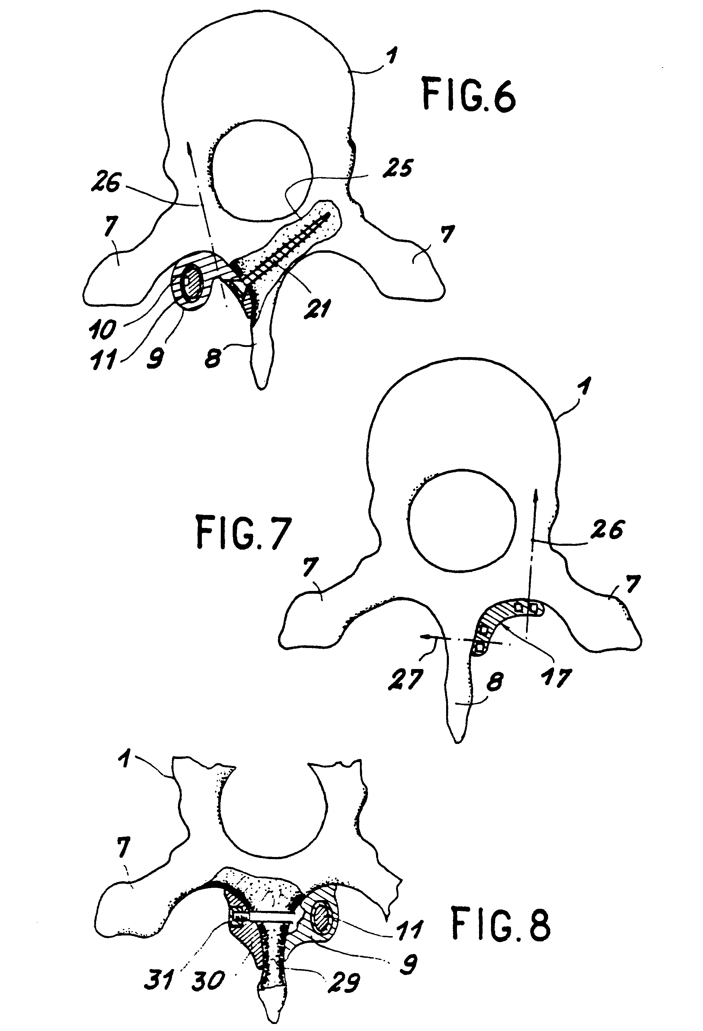

La figure 4 représente en détail à une échelle plus grande, l'une des brides de retenue 17 de la figure 3, dont la courbure caractéristique de la partie dorsale 18 est prévue pour correspondre à la concavité de la zone épineuse-lame de chaque vertèbre; la pièce 17 comporte quatre orifices tels que 19, de section carrée et de forme évasée à leur entrée supérieure et inférieure comme on le verra sur la figure 5 suivante, pour permettre une certaine flexion des tiges élémentaires 13 à 16 autour de chaque bride de retenue 17. Le dessin de la figure 4 est fait en coupe par le milieu de l'épaisseur de la bride 17 montrant ainsi l'orifice 20 réservé au vissage dans la vertèbre correspondante à l'aide d'une vis 21 munie d'une tête 22 qui vient se loger dans l'orifice 20 de cette bride de retenue 17.

Sur la figure 5, on a représenté en coupe selon la ligne XX de la figure 4, l'un des trous 19 de passage des tiges élémentaires 13 a 16 dont on voit la forme évasée sous forme de plans inclinés 23 qui permettent à l'entrée comme à la sortie de chaque trou carré 19 une certaine flexion de la tige de rappel élastique correspondante.

Les figures 6 à 8 montrent différentes formes ainsi que différentes possibilités de fixation d'une bride de retenue sur la vertèbre correspondante 1.

La figure 6 correspond au mode de mise en oeuvre de la figure 1, à l'aide d'une tige élastique unique 11, et d'une bride de retenue 9; cette dernière bride 9 est fixée dans la lame vertébrale 25 à l'aide d'une vis 21. Dans une variante, on a représenté en traits mixtes sous forme schématique, une autre implantation possible de la vis 21 en 26 dans le pédicule vertébral.

Sur la figure 7 la bride de retenue 17 qui correspond au mode de réalisation à quatre tiges élémentaires de la figure 3, peut être fixée à volonté par des vis tels que 26 et/ou 27 qui sont vissées soit dans le pédicule vertébral soit en travers de l'apophyse épineuse.

Sur la figure 8, enfin, on retrouve un mode de mise en oeuvre possédant une tige élastique unique 11 et une bride de retenue 9 qui est fixée par vissage à l'aide de la vis 28 au travers de l'apophyse épineuse 29 à l'aide d'un flasque d'appui 30, situé dans l'angle opposé lame-épineuse, et serré et bloqué en rotation par un écrou 31.

D'une façon générale, ces différents modes de fixation qui peuvent être à volonté d'ailleurs employés simultanément, sont laissés à la disposition du chirurgien qui saura, en tant qu'homme de métier, utiliser toutes les ressources de la technique connue et choisir la meilleure solution dans chaque cas particulier.

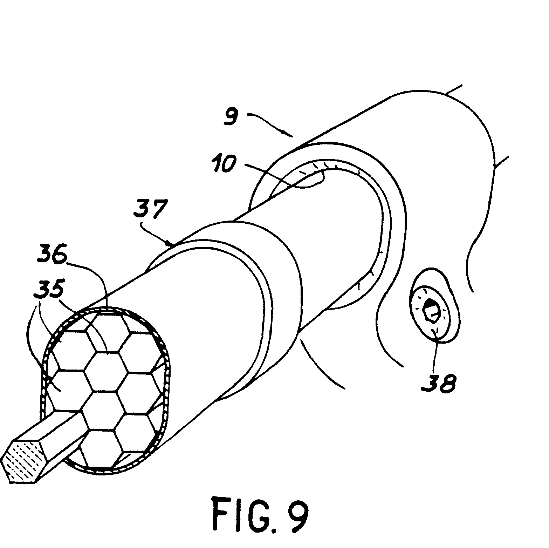

La figure 9 montre enfin un autre mode de réalisation de la structure élastique de rappel de l'appareil de correction objet de l'invention, qui comporte un certain nombre de tiges de rappel élémentaires 35 de section polyédrique, enfermées dans une gaine 36 et maintenues en position par des bagues de maintien telles que 37. Comme dans les réalisations antérieures, une bride de retenue 9 entoure la gaine 36 et est fixée avec une vis schématiquement représentée en 38 sur la vertèbre correspondantes.

Dans le cas particulier mais non limitatif de la figure 9, la réalisation représentée pour cette structure élastique de rappel comporte dix tiges élémentaires 35 de forme hexagonale pour remplir l'intérieur de la gaine 36; on peut envisager, pour perfectionner ce système, de prévoir des évidements sur le pourtour des faces latérales des tiges élémentaires 35 de façon à diminuer le coefficient de frottement entre deux tiges voisines, rendant ainsi l'ensemble de la structure élastique plus souple.

Ce mode de mise en oeuvre est très intéressant car il permet de constituer, à l'aide de tiges élémentaires facilement réalisables, un ensemble de rappel élastique de plus grande dimension pouvant être également préformé le cas échéant et capable également de produire des forces et des couples de rappel ayant l'intensité souhaitable vis-à-vis de la finalité recherchée.

Dans toutes les réalisations précédentes, on peut utiliser soit une structure élastique de rappel ayant une forme rectiligne, auquel cas on se contente de corriger les rotations et les flexions du rachis dans un plan frontal; on peut également si le matériau bio-compatible utilisé pour la structure de rappel le permet, donner à celle-ci une préformation au profil d'un rachis normal permettant ainsi à l'appareil de correction d'agir en même temps si nécessaire sur le profil rachidien dans un plan frontal.

La pose par voie chirurgicale de l'appareil de correction dynamique objet de l'invention a lieu de la façon suivante. Le patient est d'abord placé en décubitus ventral sur une table d'intervention ordinaire. Le chirurgien aborde les deux fosses épineuses et dégage le rachis jusqu'aux apophyses articulaires. Il effectue ensuite les perforations nécessaires, soit de la lame soit de l'apophyse épineuse à l'emportepièce sur le segment rachidien déformé. Une tige élastique de taille appropriée et possédant en mémoire la forme de la partie correspondante d'un rachis sain, est alors le cas échéant mise à la taille appropriée sur le segment malade intéressé. On place alors les différentes brides de retenue sur la tige et l'on présente celle-ci en la maintenant déformée selon le rachis du malade traité au moyen de trois pinces spéciales. Le chirurgien passe alors les tiges filetées dans les orifices ou les visse provisoirement selon le mode de montage choisi et met en place également le cas échéant les écrous et les flasques d'appui. Une fois le serrage de l'ensemble effectué, on peut relâcher les pinces et l'incision est alors fermée avec un drainage.

Le lever du malade doit être immédiat et puisqu'il s'agit nécessairement d'un enfant en cours de croissance, on peut prévoir la reprise de la scolarité dès la cicatrisation puisque la pose de cet appareil de correction ne conduit à aucun traumatisme interne. Il est par ailleurs conseillé pour l'opéré de porter un lombostat de façon quasi permanente durant tout le temps du séjour de l'appareil de correction sur le rachis afin de limiter l'usure des pièces en présence du fait du jeu de la tige élastique dans les différentes brides de retenue.

En fin de croissance, l'appareil de correction interne implanté, qui est devenu inutile, doit être retiré.

On remarquera enfin que le jeu de la structure de rappel élastique dans les différentes brides de retenue 9, 17 permet la croissance de l'enfant de façon normale et donne à l'ensemble de l'appareil la souplesse qu'exige la correction progressive de la déformation. L'expérience accumulée montre que l'on peut tolérer une croissance rachidienne de 5 cm sans qu'il soit nécessaire de changer l'appareil de correction pour le remplacer par un autre d'une taille supérieure. Ceci est également un avantage non négligeable de la présente invention puisqu'en moyenne, cela correspond à une augmentation de taille globale de 20 cm de l'individu.

## Revendications

1. Appareil de correction dynamique des déformations rachidiennes, comprenant une structure flexible en matériau biocompatible de forme générale allongée et destinée à être implantée le long de la partie du rachis que l'on cherche à corriger, caractérisée en ce que cette structure flexible est une structure élastique de rappel ayant au repos la forme de la partie correspondante d'un rachis et apte à exercer, lorsqu'on l'implante sur la partie malade du rachis, une correction d'intensité légère et constante dans les trois directions de l'espace, à savoir une torsion dans un plan frontal, une torsion dans un plan saggital et un couple de torsion selon l'axe de la colonne, ladite structure étant composée d'au moins une tige élastique, fixable sur chaque vertèbre (1, 2, 3, 4) de la partie malade à l'aide de brides de retenue (9, 17) vissables dans ces mêmes vertèbres (1, 2, 3, 4) dans l'angle formé par l'apophyse épineuse (8) et la lame, chaque bride de retenue (9, 17) comportant au moins un orifice de guidage (10, 19) la traversant de part en part parallèlement à l'axe du rachis, ladite tige élastique étant enfilée dans les orifices de guidage (10, 19) et immobilisée en rotation dans chaque orifice de guidage et en translation sur une seule des brides de retenue.

2. Appareil de correction dynamique des déformations rachidiennes selon la revendication 1, caractérisé en ce que chaque bride de retenue est fixée à la vertèbre associée par vissage direct dans la lame contro-latérale.

3. Appareil de correction dynamique des déformations rachidiennes selon la revendication 1, caractérisé en ce que chaque bride de retenue (9, 17) est fixée à la vertèbre à l'aide d'une vis (21, 26, 27, 28) traversant l'apophyse épineuse et serrée sur l'autre face de celle-ci par un écrou d'application d'un flasque d'appui.

4. Appareil de correction dynamique des déformations rachidiennes selon la revendication 1, caractérisé en ce que chaque bride de retenue (9, 17) est fixée à la vertèbre à l'aide d'une vis (26) vissée dans le pédicule vertébral.

5. Appareil de correction dynamique des déformations rachidiennes selon l'une quelconque des revendications 1 à 4, caractérisé en ce que la section droite des orifices de guidage (10, 19) des brides de retenue (9, 17) a une forme excluant la rotation de la tige élastique autour de son axe et choisi notamment dans le groupe comprenant les ovales, les polyèdres, les rails et les croix.

6. Appareil de correction dynamique des déformations rachidiennes selon les revendications 1 à 5 précédéntes, caractérisé en ce que les orifices (10, 19) traversant les brides de retenue ont, à leur entrée comme à leur sortie, une forme évasée permettant la flexion de la structure élastique de rappel (11, 13, 14, 15, 16) entre les points de guidage.

7. Appareil de correction dynamique des déformations rachidiennes selon les revendications 1 à 6, précédéntes, caractérisé en ce que les orifices de guidage ont une section droite ovale, dont le grand axe est contenu dans un plan sagittal.

**Patentansprüche**

1. Vorrichtung für die dynamische Korrektur von Rückgratsmißgestaltungen, mit einem flexiblen Gebilde aus gewebeverträglichem Werkstoff und von insgesamt langgestreckter Form für die Implantation entlang dem zu korrigierenden Abschnitt des Rückgrats, dadurch gekennzeichnet, daß das flexible Gebilde ein ein eine elastische Rückstellfähigkeit aufweisendes Gebilde ist, welches im Ruhezustand die Form des entsprechenden Abschnitts eines Rückgrats aufweist und bei Implantation am erkrankten Abschnitt des Rückgrats in der Lage ist, eine leichte und konstante Korrekturkraft in drei räumlichen Dimensionen auszuüben, namentlich eine Drehkraft in einer Frontalebene, eine Drehkraft in einer felgenrechten Ebene und ein Drehmoment in bezug auf die Achse der Wirbelsäule, wobei das genannte Gebilde wenigstens einen elastischen Stab aufweist, welcher mit Hilfe von Haltelaschen (9, 17), welche in dem zwischen dem Dornfortsatz (8) und dem seitlichen Fortsatz gebildeten Winkel an den einzelnen Wirbeln (1, 2, 3, 4) anschraubbar sind, an sämtlichen Wirbeln (1, 2, 3, 4) des erkrankten Abschnitts befestigbar ist, wobei jede Haltelasche (9, 17) wenigstens eine sie parallel zur Achse des Rückgrats durchsetzende Führungsöffnung (10, 19) aufweist und der elastische Stab gegen Verdrehung gesichert durch die Führungsöffnungen hindurchgeführt und in einer Haltelasche gegen Längsverschiebung gesichert ist.

2. Vorrichtung für die dynamische Korrektur von Rückgratsmißgestaltungen nach Anspruch 1, dadurch gekennzeichnet, daß jede Haltelasche durch direkte Verschraubung am seitlichen Ansatz des Dornfortsatzes jedes Wirbels befestigt ist.

3. Vorrichtung für die dynamische Korrektur von Rückgratsmißgestaltungen nach Anspruch 1, dadurch gekennzeichnet, daß jede Haltelasche (9, 17) mittels einer den Dornfortsatz durchsetzenden Schraube (21, 26, 27, 28) und jeweils einer unter Zwischenlage einer Druckscheibe an der anderen Seite des Dornfortsatzes aufgeschraubten Mutter am jeweiligen Wirbel befestigt ist.

4. Vorrichtung für die dynamische Korrektur von Rückgratsmißgestaltungen nach Anspruch 1, dadurch gekennzeichnet, daß jede Haltelasche (19, 17) mittels einer in den Ansatz des Dornfortsatzes geschraubten Schraube (27) am jeweiligen Wirbel befertigt ist.

5. Vorrichtung für die dynamische Korrektur von Rückgratsmißgestaltungen nach einem der Ansprüche 1 bis 4 dadurch gekennzeichnet, daß der Querschnitt der Führungsöffnungen (10, 19) der Haltelaschen (9, 17) eine die Verdrehung des elastischen Stabs um seine Achse ausschließende Form aufweist, insbesondere eine ovale, vieleckige, rechteckige oder kreuzförmige Gestalt.

6. Vorrichtung für die dynamische Korrektur von Rückgratsmißgestaltungen nach einem der vorstehenden Ansprüche 1 bis 5, dadurch gekennzeichnet, daß die die Haltelaschen durchsetzenden Öffnungen (10, 19) an ihrer Eintritts- sowie an ihrer Austrittsseite eine trichterförmige Gestalt aufweisen, welche eine Biegung des elastischen Rückstellgebildes (11, 13, 15, 16) zwischen den Führungspunkten ermöglicht.

7. Vorrichtung für die dynamische Korrektur von Rückgratsmißgestaltungen nach den vorstehenden Ansprüchen 1 bis 6, dadurch gekennzeichnet, daß die Führungsöffnungen eine ovale Querschnittsform haben, deren lange Achse in der felgenrechten Ebene verläuft.

**Claims**

1. Appliance for the dynamic correction of rachidial deformities comprising a flexible structure made from a biocompatible material and having a generally elongated shape and which is implanted along that part of the rachis which it is wished to correct, characterized in that said flexible structure is an elastic restoring structure having at rest the shape of the corresponding part of a rachis and which is able to exert, when implanted on the diseased part of the rachis, a correction with a slight, constant intensity in the three directions in space, namely a torsion in a frontal plane, a torsion in a sagittal plane and a torque along the axis of the column, said structure being constituted by at least one elastic rod fixable to each vertebra (1, 2, 3, 4) of the diseased part with the aid of retaining clamps (9, 17) which can be screwed into the said vertebrae (1, 2, 3, 4) in the angle formed by the spinous process (8) and the plate, each retaining clamp (9, 17) having at least one guide opening (10, 19) passing through the same parallel to the axis of the rachis, said elastic rod being introduced into the guide openings (10, 19) and fixed in rotation in each guide opening and in translation on a single retaining clamp.

2. Appliance for the dynamic correction of rachidial deformities according to claim 1, characterized in that each retaining clamp is fixed to the associated vertebra by direct screwing into the centro-lateral disk.

3. Appliance for the dynamic correction of rachidial deformities according to claim 1, characterized in that each retaining clamp (9, 17) is fixed to the vertebra with the aid of a screw (21, 26, 27, 28) which passes through the spinous process and is fixed to the other face therof by a nut for applying a bearing plate.

4. Appliance for the dynamic connection of rachidial deformities according to claim 1, characterized in that each retaining clamp is fixed to the vertebra with the aid of a screw, which is screwed into the vertebral pedicle.

5. Appliance for the dynamic correction of rachidial deformities according to any one of the claims 1 to 4, characterized in that the cross-section of the guidance openings (10, 19) of the retaining flanges (9, 17) have a shape excluding the rotation of the elastic rod about its axis and more particularly chosen from oval or polyhedral shapes and rails and crosses.

6. Appliance for the dynamic correction of rachidial deformities according to claims 1 to 5, characterized in that the openings (10, 19) passing through the retaining clamps have, at their inlet and outlet, a flared or widened shape permitting the bending of the elastic restoring structure (11, 13, 14, 15, 16) between the guidance points.

7. Appliance for the dynamic correction of rachidial deformities according to claims 1 to 6, characterized in that the guidance openings have an oval cross-section, whereof the major axis is contained in a sagittal plane.

# FIG.1

# FIG.2

FIG. 3

FIG. 4

FIG. 5

FIG.6

FIG.7

FIG.8

FIG. 9